# EUROPEAN PATENT APPLICATION

(11) **EP 4 613 874 A1**
(43) Date of publication of application: **10.09.2025**
(21) Application number: 22969720.6
(22) Date of filing: 29.12.2022
(51) Int. Cl.: C12Q 1/6855, C12Q 1/6869, G01N 27/403

(54) **SEQUENCING LINKER, SEQUENCING LINKER COMPLEX, METHOD FOR MULTIPLE AMPLIFICATIONS OF TARGET NUCLEIC ACID SEQUENCE, AND METHOD FOR NANOPORE SEQUENCING**

(71) Applicant: BGI Shenzhen, Shenzhen, Guangdong 518083 (CN); BGI Shenzhen Co., Ltd, Shenzhen, Guangdong 518083 (CN)
(72) Inventor: CHEN, Junyi, Shenzhen, Guangdong 518083 (CN); JI, Zhouxiang, Shenzhen, Guangdong 518083 (CN); DONG, Yuliang, Shenzhen, Guangdong 518083 (CN); WANG, Ou, Shenzhen, Guangdong 518083 (CN); ZENG, Tao, Shenzhen, Guangdong 518083 (CN); LI, Yuxiang, Shenzhen, Guangdong 518083 (CN); ZHANG, Wenwei, Shenzhen, Guangdong 518083 (CN); XU, Xun, Shenzhen, Guangdong 518083 (CN)
(74) Representative: Zacco GmbH
(86) International application number: PCT/CN2022/143629
(87) International publication number: WO 2024/138573

(57) **Abstract**

Disclosed in the present disclosure are a sequencing adapter, a sequencing adapter complex, a method for multiple amplifications of a target nucleic acid sequence, and a method for nanopore sequencing. **The** sequencing adapter includes a first strand and a second strand. **The** first strand sequentially includes, from the 5' end to the 3' end, a sequencing guide sequence, a helicase binding sequence, a restriction structure, a first complementary sequence, and a first primer sequence; the restriction structure prevents a helicase from moving; the first strand has a 3' free end; the second strand sequentially includes, from the 5' end to the 3' end, a second complementary sequence, and a constraining sequence or a sequence complementary to the nucleic acid sequence of the constraining sequence; the constraining sequence includes the nucleic acid sequence modified with a hydrophobic molecule at an end; the first complementary sequence is reversely complementary to the second complementary sequence, and the sequencing adapter is formed by annealing the first strand and the second strand; and the sequencing adapter further includes a structure that prevents the second strand from being displaced by a polymerase. **The** sequencing adapter and sequencing adapter complex of the present disclosure are simple in construction process, and high in yield.

## Description

### Technical Field

The present disclosure relates to the technical field of sequencing, and specifically, to a sequencing adapter, a sequencing adapter complex, a method for multiple amplifications of a target nucleic acid sequence, and a method for nanopore sequencing.

### Background

A nanopore sequencing technology is a single-molecule level detection technology characterized by rapid sequencing speed, long read lengths, direct sequencing capability, high throughput, low cost, compact size and portability, etc. During nanopore sequencing, individual nanopores are embedded in an insulating impermeable membrane to form a stable ion current channel, and single-stranded nucleic acid molecules pass through the nanopores under an applied voltage, thereby reducing ion currents passing through the nanopores. Since different bases on the single-stranded nucleic acid molecule have different structures, molecular weights, volumes, etc., the currents passing through the nanopores show differences corresponding to base sequences. A current change signal is analyzed by using a deep learning algorithm, so as to read a pore-passing single-stranded nucleic acid sequence in real time. However, the existing nanopore sequencing technology is still limited in accuracy, which restricts an application range of the nanopore sequencing technology.

For example, a target nucleic acid sequence 4-times nanopore sequencing technology developed by Oxford Nanopore Technologies (ONT) includes: (1) double-stranded ends of a nucleic acid to be tested are connected to different sequencing adapters, one end is a Y-shaped adapter binding to a helicase and a polymerase, and one end is a hairpin structure adapter. A top strand of the Y-shaped adapter is composed of a guide sequence, a helicase binding site, a spacer region, and a sequence complementary to a bottom strand, and the bottom strand of the adapter is composed of a sequence complementary to the top strand and a sequence containing a hairpin structure. (2) The helicase is bound between the guide sequence and the spacer region of the top strand of the Y-shaped adapter, and is blocked by the spacer region; and the polymerase is bound to a hairpin position of the Y-shaped adapter, and amplification is performed by using dNTP, so as to cause sense and antisense strands of the nucleic acid to be tested to be both amplified once. (3) Amplification products are added to a sequencing system embedded with nanopores, the guide sequence is captured by the nanopores, then the helicase passes through the spacer region under the action of an electric field force, and nanopore sequencing is controlled, such that the target nucleic acid sequence may be subjected to 4 times of sequencing.

The 4-times nanopore sequencing technology developed by the ONT has the following disadvantages. (1) connecting different sequencing adapters to the double-stranded ends of the nucleic acid to be tested is a complex problem, a complex preliminary operation (e.g., by means of PCR, the method for connecting the adapters to the two ends is introduced into a restriction enzyme site, different sticky ends are obtained through enzyme digestion, and then connection is performed with an adapter having different sticky ends; or different adapters are added at the same time during TA ligation, and then a target product of which two ends are connected to different adapters is obtained through filtration) is required, and this process is complex in operation and low in product obtaining efficiency. (2) By using the method, only up to 4 times of sequencing are performed on the target nucleic acid sequence, and the number of times for sequencing a target nucleic acid fragment is limited. (3) When the method is used for performing 4 times of sequencing, the helicase controls the sense strand, the antisense strand, the sense strand (amplification), and the antisense strand (amplification) sequentially pass through the nanopore to complete sequencing. However, sequencing speeds of different strands in such sequencing process are significantly different, this is because there is a difference in the speeds of the same helicase controlling double strands for sequencing and controlling a single strand for sequencing, and this 4-times sequencing method inevitably needs to control the double strands for sequencing and also control the single strand for sequencing, which will increase the difficulty of base recognition during sequencing result analysis, thus ultimately affecting sequencing accuracy.

### Summary

The present disclosure is intended to provide a sequencing adapter, a sequencing adapter complex, a method for multiple amplifications of a target nucleic acid sequence, and a method for nanopore sequencing, so as to improve sequencing efficiency and accuracy.

In order to realize the above objective, an aspect of the present disclosure provides a sequencing adapter. The sequencing adapter includes a first strand and a second strand. The first strand includes a sequencing guide sequence, a helicase binding sequence, a restriction structure, a first complementary sequence, and a first primer sequence, which are sequentially connected from a 5' end to a 3' end; the restriction structure prevents a helicase from moving; the first strand has a 3' free end; the second strand includes a second complementary sequence, and a constraining sequence or a sequence complementary to a nucleic acid sequence of the constraining sequence, which are sequentially connected from the 5' end to the 3' end; the constraining sequence includes the nucleic acid sequence modified with a hydrophobic molecule at an end; the first complementary sequence is reversely complementary to the second complementary sequence, and the sequencing adapter is formed by annealing the first strand and the second strand; and the sequencing adapter further includes a structure that prevents the second strand from being displaced by a polymerase.

Further, the structure that prevents the second strand from being displaced by the polymerase includes at least one of the following: a sequence that is located on the 5' end of the second strand and inhibits polymerase strand displacement activity, or a blocking structure located between the first complementary sequence and the first primer sequence of the first strand, where the blocking structure prevents the nucleic acid sequence from extending through the polymerase.

Further, the sequence that is located on the 5' end of the second strand and inhibits the polymerase strand displacement activity includes an artificially modified nucleic acid sequence, and an artificially modified nucleotide in the artificially modified nucleic acid sequence includes at least one of LNA, PNA, or BNA.

Further, the artificially modified nucleic acid sequence includes 2-10 artificially modified nucleotides.

And/or, the blocking structure includes at least one of a spacer region or an abasic site, the spacer region includes a spacer, and the spacer is selected from at least one of iSp18, iSp9, iSpC3, iSpC6, or iSpC12.

Further, the blocking structure includes 1-10 spacers or abasic sites.

Further, the restriction structure includes a spacer region, the spacer region includes a spacer, and the spacer is selected from at least one of iSp18, iSp9, iSpC3, iSpC6, or iSpC12; the restriction structure includes 2-8 spacers.

Preferably, the sequencing guide sequence includes 10-50 nucleotides or iSpC3.

Preferably, the helicase binding sequence includes 5-40 nucleotides, more preferably, the helicase binding sequence includes 5-40 thymidylic acids.

Preferably, the first complementary sequence or the second complementary sequence includes 5-80 nucleotides.

Preferably, the nucleic acid sequence of the constraining sequence includes 10-50 nucleotides.

Further, the hydrophobic molecule is selected from any one or more of the following: a lipid, a fatty acid, a sterol, a carbon nanotube, a peptide, a protein, and an amino acid, for example, cholesterol, palmitate, or tocopherol.

Further, the first primer sequence includes a specific primer or a random primer, and the first primer sequence is able to bind to a circular library.

Preferably, the first primer sequence includes 5-80 nucleotides.

Optionally, the circular library is a single-stranded circular library or a double-stranded circular library.

Optionally, the single-stranded circular library has an introduced known sequence, and the known sequence complementarily binds to the first primer sequence.

Optionally, the double-stranded circular library has an introduced known sequence, and there is a bulge or gap at a complementary junction of the known sequence and the first primer sequence.

Optionally, there is no known sequence in the circular library, the first primer sequence is a degenerate primer sequence, and any sequence of the circular library complementarily binds to the degenerate primer sequence.

Another aspect of the present disclosure provides a sequencing adapter complex. **The** sequencing adapter complex includes the sequencing adapter and a helicase. **The** helicase binds to a helicase binding sequence, and unwinds in a 5'-3' direction; preferably, the helicase is selected from any one or more of the following: a Dda helicase, a Pif 1 helicase, an XPD helicase, a T7Gp41 helicase, and a DnaB helicase; and the helicase is subjected to mutation modification. Mutation modification mainly refers to a mutation that improves sequencing performance.

Another aspect of the present disclosure provides a kit for multiple amplifications of a target nucleic acid sequence. The kit includes any one of the above sequencing adapters.

Further, the kit further includes a polymerase having strand displacement activity, a reaction buffer, and a helicase.

Another aspect of the present disclosure provides a kit for multiple amplifications of a target nucleic acid sequence. The kit includes any one of the above sequencing adapter complexes.

Further, the kit further includes a polymerase having strand displacement activity and a reaction buffer.

Further, the kit further includes one or more of a reverse primer, dNTP or NTP, and a circular library construction-related reagent.

The reverse primer is able to complementarily bind to a single strand obtained by rolling circle amplification of a circular library; the reverse primer has a 3' free end; and preferably, a 5' end of the reverse primer contains an artificially modified nucleic acid sequence that is able to inhibit polymerase strand displacement activity.

Preferably, an artificially modified nucleotide in the artificially modified nucleic acid sequence is one or more of LNA, PNA, and BNA; and more preferably, the number of the artificially modified nucleotides in the artificially modified nucleic acid sequence is 2-10.

Another aspect of the present disclosure provides a method for multiple amplifications of a target nucleic acid sequence. The method includes the following steps.

At S1, the sequencing adapter complex, a target nucleic acid sequence circular library, and a polymerase having strand displacement activity are bound.

At S2, rolling circle amplification is performed on the circular library, and a reverse primer is introduced simultaneously, where the reverse primer and a first primer sequence are reversely complementary to each other, and complementarily bind to a single strand obtained by rolling circle amplification of the circular library, so as to realize rolling circle amplification and two-strand synthesis of the target nucleic acid sequence circular library, thereby completing multiple amplifications of a target nucleic acid sequence.

Further, the reverse primer is able to complementarily bind to a single strand obtained by rolling circle amplification of a circular library; the reverse primer has a 3' free end; and a 5' end of the reverse primer contains an artificially modified nucleic acid sequence that is able to inhibit polymerase strand displacement activity.

Preferably, an artificially modified nucleotide in the artificially modified nucleic acid sequence is one or more of LNA, PNA, and BNA; and more preferably, the number of the artificially modified nucleotides in the artificially modified nucleic acid sequence is 2-10.

Preferably, the reverse primer is added in excess.

Further, S1 includes: a first strand of the sequencing adapter in the sequencing adapter complex binds to the circular library through complementary base pairing, and the polymerase uses the circular library as a template and the first strand as a primer to bind to a primer-template junction, so as to form an amplification complex.

And/or, S2 includes: the polymerase uses dNTP or NTP for a polymerization reaction, the first strand of the sequencing adapter extends to obtain a sequencing strand, based on the strand displacement activity of the polymerase, the sequencing strand extends by means of rolling circle amplification, the reverse primer specifically binds to the sequencing strand and extends under the action of the polymerase, so as to complete two-strand synthesis, and two-strand synthesis stops when encountering the artificially modified nucleic acid sequence of the 5' end of the previous reverse primer or the second strand of the sequencing adapter, or a sequence that is able to terminate polymerase amplification between a first complementary sequence and a first primer sequence of the first strand of the sequencing adapter, so as to complete multiple amplifications of the target nucleic acid sequence.

Further, a reaction temperature of S1 is 4 °C-37 °C, and a time is 10-600 minutes.

Preferably, a reaction temperature of S2 is 20 °C-37 °C, and a time is 10-360 minutes.

Preferably, the reaction temperature of S2 is 30 °C-37 °C, and the time is 20-360 minutes. Further, the polymerase having strand displacement activity is selected from a DNA polymerase or an RNA polymerase.

Optionally, the polymerase is a polymerase having strand displacement activity that is obtained by modifying a polymerase without strand displacement activity.

Preferably, the polymerase is selected from a Bst DNA polymerase, an SD DNA polymerase, a phi29 DNA polymerase, a Bsu Large Fragment DNA polymerase, a Klenow Fragment DNA polymerase, a T4 DNA polymerase, a T7 DNA polymerase, a DNA Polymerase I, a T3 RNA polymerase, a T7 RNA polymerase, an SP6 RNA polymerase, an *E.coli* RNA polymerase, or any combination thereof.

Further, S1 and S2 are performed in a reaction buffer.

The reaction buffer contains a pH buffer system, preferably, the pH buffer system is a dihydrogen phosphate-hydrogen phosphate buffer system, a carbonate-sodium bicarbonate buffer system, a Tris-HCl buffer system, a HEPES buffer system, a MOPS buffer system, or any combination thereof.

Preferably, the reaction buffer contains K⁺, Na²⁺, or any combination thereof.

Preferably, the reaction buffer contains Mg²⁺, Mo²⁺, Cu²⁺, Fe²⁺, Zn²⁺, Ca²⁺, Pb²⁺, Cd²⁺, or any combination thereof.

Preferably, the reaction buffer contains an additive or an auxiliary reagent that enhances a polymerase extension reaction, and the additive or the auxiliary reagent is dimethyl sulfoxide, glycerol, formamide, bovine serum albumin, ammonium sulphate, polyethylene glycol, gelatin, a non-ionic detergent, N,N,N-trimethylglycine, a single-stranded nucleic acid binding protein, dithiothreitol, ethylenediamine tetraacetic acid, or any combination thereof.

Another aspect of the present disclosure provides a method for nanopore sequencing. The method includes the following steps.
1) A target product after a target nucleic acid sequence is amplified is obtained by using any one of the above methods for multiple amplifications of a target nucleic acid sequence.
2) The target product binds to a membrane of a nanopore sequencer through a constraining sequence, where the target product is sequenced under control of a helicase, so as to sequence a target sequence.

Further, step 2) includes the following operations.

A second strand of a sequencing adapter binds to the constraining sequence, and an amplification product binds to the membrane of the nanopore sequencer, or the constraining sequence on the second strand of the sequencing adapter directly binds the amplification product to the membrane of the nanopore sequencer, where a sequence length of the constraining sequence is 10-50 nucleotides; an end of the constraining sequence is modified with a hydrophobic molecule, and the hydrophobic molecule is selected from any one or more of the following: a lipid, a fatty acid, a sterol, a carbon nanotube, a peptide, a protein, and an amino acid, for example, cholesterol, palmitate, or tocopherol.

A sequencing voltage is applied, then a guide sequence of the sequencing adapter is captured by a nanopore under the action of an electric field force, the helicase passes through a spacer region, and the target nucleic acid sequence is sequenced for the plurality of times under the control of the helicase.

Preferably, the sequencing voltage is more than 10 mV, preferably 50 mV-250 mV.

Further, the nanopore of the nanopore sequencer is a transmembrane protein pore or a solid-state pore.

Preferably, a transmembrane protein from which the transmembrane protein pore derives is selected from hemolysin, MspA, MspB, MspC, MspD, FraC, ClyA, PA63, CsgG, CsgD, XcpQ, SP1, a phi29 connector protein, InvG, GspD, or any combination thereof.

Optionally, the transmembrane protein is also linked to an additional peptide, and the additional peptide is selected from a tag, a restriction enzyme cutting site, a signal peptide or presequence, a detectable marker, or any combination thereof.

Optionally, the membrane of the nanopore sequencer is an amphiphilic membrane, a polymer membrane, or any combination thereof, and the membrane of the nanopore sequencer is a phospholipid bilayer, or a diblock copolymer, or a triblock copolymer.

Further, a sequencing buffer used during sequencing contains a pH buffer system, preferably, the pH buffer system is a dihydrogen phosphate-hydrogen phosphate buffer system, a carbonate-sodium bicarbonate buffer system, a Tris-HCl buffer system, a HEPES buffer system, a MOPS buffer system, or any combination thereof.

Preferably, the sequencing buffer contains NTP, dNTP, ddNTP, or any combination thereof.

Preferably, the sequencing buffer contains K⁺, Na²⁺, or any combination thereof.

Preferably, the sequencing buffer contains Mg²⁺, Mo²⁺, Cu²⁺, Fe²⁺, Zn²⁺, Ca²⁺, Pb²⁺, Cd²⁺, or any combination thereof.

There is a sequence complementary to the circular library in the sequencing adapter of the present disclosure. The polymerase having strand displacement activity uses the circular library as the template and the sequencing adapter complex as the primer, and the sequencing strand is obtained through rolling circle amplification. Excess reverse primers continuously bind to the sequencing strand obtained through rolling circle amplification, and two-strand synthesis is continuously completed under the action of the polymerase. The 5' end of the reverse primer has the artificially modified nucleic acid sequence that can inhibit the strand displacement activity of the polymerase. The sequencing adapter further includes the artificially modified nucleic acid sequence inhibiting the strand displacement activity of the polymerase on the 5' end of the second strand, and/or the sequence stopping polymerase amplification between the first complementary sequence and the first primer sequence of the first strand. The sequencing adapter pre-binds to the helicase to construct the sequencing adapter complex, and nanopore sequencing may be directly performed after amplification is completed, thereby sequencing the target nucleic acid for the plurality of times.

By using the technical solutions of the present disclosure, at least the following beneficial effects are achieved. 1) the sequencing adapter and the sequencing adapter complex used in the present disclosure are simple in construction process and high in yield, the sequencing adapter is formed by annealing two strands, and the sequencing adapter complex is formed by combining the well-annealed sequencing adapter to the helicase; 2) the present disclosure is suitable for the single-stranded circular library constructed by various manners, and is also suitable for the double-stranded circular library that has bulges or gaps at the junction complementary to the first primer sequence of the sequencing adapter; 3) during the amplification process used in the present disclosure, the generation of long single strands may be reduced by adding excess reverse primers, thereby reducing the generation of a complex secondary structure affecting sequencing; 4) in the present disclosure, the target nucleic acid sequence is amplified for the plurality of times to obtain the sequencing library, such that the construction process is simple, and the yield is high; and the 5' end of the reverse primer has the artificially modified nucleic acid sequence that can inhibit the strand displacement activity of the polymerase, the 5' end of the second strand of the sequencing adapter has the artificially modified nucleic acid sequence inhibiting the strand displacement activity of the polymerase, and/or the sequence stopping polymerase amplification between the first complementary sequence and the first primer sequence of the first strand, thereby preventing the polymerase from displacing the previously synthesized double-strand or the second strand of the sequencing adapter; 5) in the present disclosure, the sequencing strand has bound to the helicase after amplification is completed, such that ligation of helicase-incorporated adapters becomes unnecessary, and ligation efficiency does not need to be considered, thereby achieving high utilization efficiency of the amplification product; and 6) in the present disclosure, nanopore sequencing may be performed on the target nucleic acid sequence for the plurality of times, the sequencing process remains under helicase-controlled double-stranded mode, speeds for multiple sequencing are relatively the same, such that data analysis difficulty is reduced, and sequencing accuracy is improved.

### Brief Description of the Drawings

The drawings, which form a part of the present disclosure, are used to provide a further understanding of the present disclosure. The exemplary embodiments of the present disclosure and the description thereof are used to explain the present disclosure, but do not constitute improper limitations to the present disclosure. In the drawings:
Fig. 1 is a schematic diagram of a method for multiple amplifications of a target nucleic acid sequence and for nanopore sequencing according to an implementation of the present disclosure.
Fig. 2 is a schematic diagram of construction of a sequencing adapter and a sequencing adapter complex according to Embodiment 2.
Fig. 3 is an electrophoretic detection diagram of construction of a sequencing adapter and a sequencing adapter complex according to Embodiment 2.
Fig. 4 is a current signal diagram of nanopore sequencing of a sequencing adapter in a control group according to Embodiment 3.
Fig. 5 is a current signal diagram of multiple nanopore sequencing of a target nucleic acid sequence to be tested in an experimental group according to Embodiment 3.
Fig. 6 is an enlarged view of a current signal of a signature sequence in Fig. 5.
Fig. 7 is an enlarged view of a current signal of a hairpin sequence in Fig. 5.
Fig. 8 is a schematic diagram (double-stranded circular library) of a method for multiple amplifications of a target nucleic acid sequence and for nanopore sequencing according to another implementation of the present disclosure.
Fig. 9 is a schematic diagram of a method for multiple amplifications of a target nucleic acid sequence and for nanopore sequencing according to another implementation of the present disclosure (there is a sequence terminating polymerase amplification in a first strand of a sequencing adapter).

### Detailed Description of the Embodiments

It is to be noted that the embodiments in the present disclosure and the features in the embodiments may be combined with one another without conflict. The present disclosure will be described below in detail with reference to the drawings and the embodiments.

As mentioned in the Background, existing nanopore sequencing technologies still have a series of technical problems, and are still limited in accuracy, which restricts an application range of the nanopore sequencing technologies. If a target nucleic acid sequence is subjected to multiple nanopore sequencing, sequencing accuracy is significantly improved. In view of this, the present disclosure provides the following technical solutions.

A typical implementation of the present disclosure provides a sequencing adapter. The sequencing adapter includes a first strand and a second strand. The first strand includes a sequencing guide sequence, a helicase binding sequence, a restriction structure, a first complementary sequence, and a first primer sequence, which are sequentially connected from a 5' end to a 3' end; the restriction structure prevents a helicase from moving; the first strand has a 3' free end; the second strand includes a second complementary sequence, and a constraining sequence or a sequence complementary to a nucleic acid sequence of the constraining sequence, which are sequentially connected from the 5' end to the 3' end; the constraining sequence includes the nucleic acid sequence modified with a hydrophobic molecule at an end; the first complementary sequence is reversely complementary to the second complementary sequence, and the sequencing adapter is formed by annealing the first strand and the second strand; and the sequencing adapter further includes a structure that prevents the second strand from being displaced by a polymerase. The sequencing adapter of the present disclosure is simple in design, it is easy to anneal two strands as a destination configuration, and a yield is high.

It is understandable that, if the constraining sequence is on the second strand, the 3' end of the second strand is modified with the hydrophobic molecule; and if the sequence complementary to the nucleic acid sequence of the constraining sequence is on the second strand, the 5' end of the constraining sequence is modified with the hydrophobic molecule.

Another typical implementation of the present disclosure provides a sequencing adapter complex. The sequencing adapter complex includes the sequencing adapter of the present disclosure and a helicase, and the helicase binds to a helicase binding sequence of a first strand, and unwinds in a 5'-3' direction; preferably, the helicase is selected from any one or more of the following: a Dda helicase, a Pif 1 helicase, an XPD helicase, a T7 Gp41 helicase, and a DnaB helicase; and the helicase is subjected to mutation modification.

Based on the sequencing adapter complex provided in the present disclosure, the applicant further invents a method for multiple amplifications of a target nucleic acid sequence, and a method for nanopore sequencing. There is a sequence complementary to the circular library in the sequencing adapter complex of the present disclosure. A polymerase having strand displacement activity uses a circular library as a template and the sequencing adapter complex as a primer, and a sequencing strand is obtained through rolling circle amplification. Excess reverse primers continuously bind to the sequencing strand obtained through rolling circle amplification, and two-strand synthesis is continuously completed. A 5' end of the reverse primer has an artificially modified nucleic acid sequence that can inhibit the strand displacement activity of the polymerase. A 5' end of a second strand of a sequencing adapter has an artificially modified nucleic acid sequence inhibiting the strand displacement activity of the polymerase, and/or a sequence stopping polymerase amplification between a first complementary sequence and a first primer sequence of a first strand, thereby preventing the polymerase from displacing the previously synthesized double-strand or the second strand of the sequencing adapter. The sequencing adapter pre-binds to a helicase to construct the sequencing adapter complex, and nanopore sequencing may be directly performed after amplification is completed, thereby sequencing the target nucleic acid for the plurality of times.

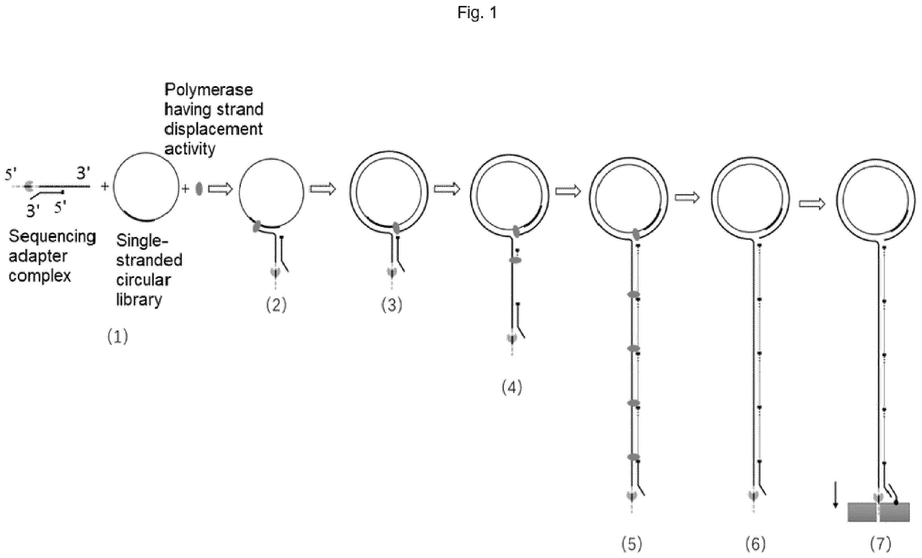

According to a preferred implementation of the present disclosure, specific processes of the methods for multiple amplifications of a target nucleic acid sequence and for nanopore sequencing may be referred to Fig. 1. In Process (1), components, which are the sequencing adapter complex, a single-stranded circular library, and the polymerase having strand displacement activity, required for sequencing are prepared. A construction process of the sequencing adapter complex includes: first, the first strand and the second strand are annealed as the sequencing adapter; the first strand sequentially includes, from 5' to 3', a guide sequence (30 iSpC3s, represented with a gray dashed), a helicase binding sequence (10 Ts, represented with a black solid line), a spacer region (4 iSp18s, represented with a gray dashed), and sequences (represented with a black solid line) complementary to a second strand complementary sequence and to the single-stranded circular library; the second strand sequentially includes, from 5' to 3', sequences complementary to a first strand complementary sequence and to a constraining sequence (a 5' end includes an artificially modified nucleic acid sequence that can inhibit polymerase strand displacement activity, for example, LNA, PNA, or BNA, etc., represented with a bold black solid line; and the remaining unmodified nucleic acid sequences are represented with black solid lines). Then, the sequencing adapter and the helicase (light gray with a notched circle, and an unwinding direction is a direction from 5' to 3') are incubated, then a reaction buffer containing ATP is added, the helicase bound to the helicase binding sequence cannot displace and remain bound to the first strand due to the action of the spacer region, and the helicase bound to the remaining sequences displaces and detaches. Finally, through magnetic bead purification, the sequencing adapter complex in which a ratio of the helicase to the sequencing adapter is 1:1 is obtained. A construction process of the single-stranded circular library includes: the single-stranded circular library is prepared by connecting two ends of a double-stranded target nucleotide to an adapter containing a hairpin structure, or the double-stranded target nucleotide is prepared into the single-stranded circular library by using a MGI Easy PCR-Free DNA library preparation kit (MGI, 1000013456), or the double-stranded target nucleotide is prepared into the single-stranded circular library by other similar methods, and the sequence (bold black solid line) complementary to the first strand of the sequencing adapter is introduced during the preparation of the single-stranded circular library. The polymerase is a polymerase having strand displacement activity (grey ellipse). In Process (2), the sequencing adapter complex, the single-stranded circular library, and the polymerase having strand displacement activity are incubated together, and the first strand of the sequencing adapter binds to the single-stranded circular library through complementary base pairing. The polymerase binds to a primer-template junction by using the circular library as a template and using the first strand as a primer, so as to form a complex. In Process (3), the polymerase undergoes a polymerization reaction by using dNTP or NTP in the reaction buffer, and the first strand extends to obtain the sequencing strand. Meanwhile, the helicase bound to the sequencing adapter cannot unwind the double-strand by using the dNTP or NTP in the reaction buffer due to the presence of the spacer region. In Process (4), since the polymerase has strand displacement activity, the sequencing strand extends by means of rolling circle amplification, and a known sequence introduced by the single-stranded circular library is also amplified by the polymerase, such that binding sites of the reverse primer (the 5' end includes the artificially modified nucleic acid sequence that can inhibit polymerase strand displacement activity, for example, LNA, PNA, or BNA, etc., represented with the bold black solid line; and the remaining unmodified nucleic acid sequences are represented with the black solid lines) are obtained continuously. The reverse primer specifically binds to the sequencing strand, and extends under the action of the polymerase. In Process (5), the sequencing strand continuously extends by means of rolling circle amplification, and excess reverse primers continuously bind to the sequencing strand, so as to continuously complete two-strand synthesis, thereby avoiding the presence of the sequencing strand in the form of a single strand (a newly-synthesized double-strand is represented with a gray solid line). During this process, since the 5' ends of the reverse primer and the second strand of the sequencing adapter complex both include the artificially modified nucleic acid sequence that can inhibit polymerase strand displacement activity, the strand displacement activity of the polymerase can be inhibited, thereby preventing the polymerase from displacing the previously synthesized double-strand or the second strand of the sequencing adapter complex. In Process (6), through magnetic bead purification, a double-stranded amplification product for nanopore sequencing is obtained. The target nucleic acid sequence is amplified for a plurality of times, and the ends of the target nucleic acids amplified for the plurality of times all have the sequencing adapters bound to the helicase. In Process (7), the constraining sequence (the 5' end being modified with cholesterol, and represented with a black circle) binds to the second strand of the sequencing adapter, and the amplification product binds to a membrane material (the membrane is represented with a gray square, and a nanopore is represented with a white channel) near the nanopore. A sequencing voltage is applied, then a guide sequence of the sequencing adapter is captured by a nanopore under the action of an electric field force (represented with a black arrow), and the target nucleic acid sequence is sequenced for the plurality of times under the control of the helicase.

The advantages of this design are as follows. ① The sequencing adapter and the sequencing adapter complex are simple in construction process and high in yield, the sequencing adapter is formed by annealing two strands, and the sequencing adapter complex is formed by combining the well-annealed sequencing adapter to the helicase; ② the present disclosure is suitable for the single-stranded circular library constructed by various manners, and is also suitable for the double-stranded circular library that has bulges or gaps at the junction complementary to the first primer sequence of the sequencing adapter; ③ excess reverse primers are added during the amplification process, the generation of long single strands is reduced, thereby reducing the generation of a complex secondary structure affecting sequencing; ④ in the present disclosure, the target nucleic acid sequence is amplified for the plurality of times to obtain the sequencing library, such that the construction process is simple, and the yield is high; and the 5' end of the reverse primer has the artificially modified nucleic acid sequence that can inhibit the strand displacement activity of the polymerase, the 5' end of the second strand of the sequencing adapter has the artificially modified nucleic acid sequence inhibiting the strand displacement activity of the polymerase, and/or the sequence stopping polymerase amplification between the first complementary sequence and the first primer sequence of the first strand, thereby preventing the polymerase from displacing the previously synthesized double-strand or the second strand of the sequencing adapter; ⑤ the sequencing strand has bound to the helicase after amplification is completed, such that ligation of helicase-incorporated adapters becomes unnecessary, and ligation efficiency does not need to be considered, thereby achieving high utilization efficiency of the amplification product; and ⑥ nanopore sequencing may be performed on the target nucleic acid sequence for the plurality of times, the sequencing process remains under helicase-controlled double-stranded mode, durations and sequencing speeds for multiple sequencing are relatively the same, such that data analysis difficulty is reduced, and sequencing accuracy is improved.

Based on the inventive conception of the present disclosure, some of the technical features in the above preferred implementations are variable or replaceable. For example, in some implementation solutions, preferably, the guide sequence includes 10-50 nucleotides or iSpC3; preferably, the helicase binding sequence of the first strand includes 5-40 nucleotides, more preferably, the helicase binding sequence of the first strand includes 5-40 thymidylic acids (T); preferably, the spacer region of the first strand includes 2-8 spacer modifications, such as iSp18, iSp9, iSpC3, iSpC6, iSpC12, or any combination thereof; preferably, a length of a sequence of the first strand that is complementary to the second strand is 5-80 nucleotides; preferably, a length of a sequence of the first strand that is complementary to the circular library is 5-80 nucleotides; preferably, a length of a sequence of the second strand that is complementary to the first strand is 5-80 nucleotides; and preferably, a length of a sequence of the second strand that is complementary to the constraining sequence is 10-50 nucleotides.

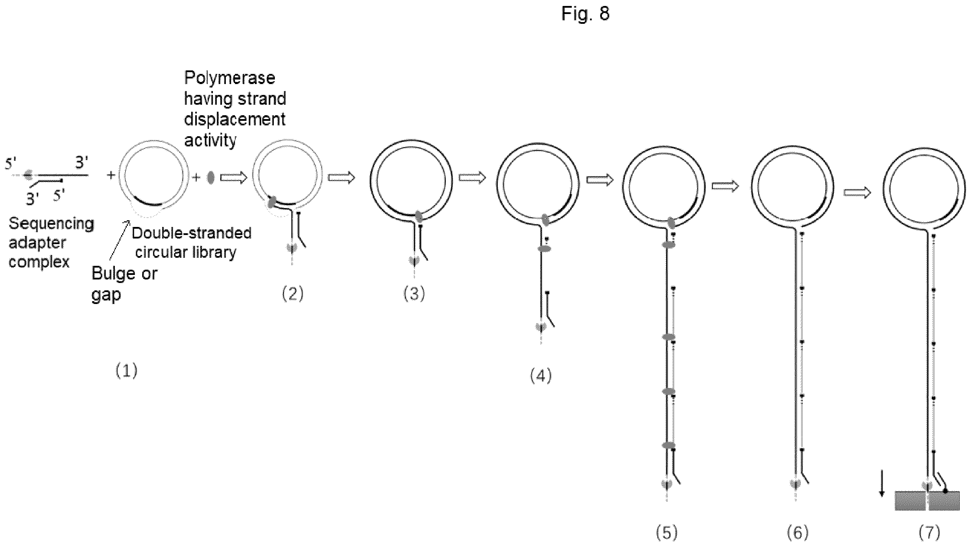

In some implementation solutions, the technical solution of the present disclosure may select a double-stranded circular library. There are bulges or gaps at a junction complementary to the first primer sequence of the first strand of the sequencing adapter. Details on how to realize multiple amplification on the target nucleic acid sequence and nanopore sequencing are referred to Fig. 8.

In some implementation solutions, the technical solution of the present disclosure may select a circular library. There is no known sequence in the circular library, the first primer sequence of the first strand is a degenerate primer sequence, and any sequence of the circular library complementarily binds to the degenerate primer sequence.

In some implementation solutions, the artificially modified nucleic acid sequence that can inhibit polymerase strand displacement activity located on the 5' end of the second strand is LNA, PNA, BNA, or any combination thereof; and the number of sequences that can inhibit polymerase strand displacement activity is 2-10.

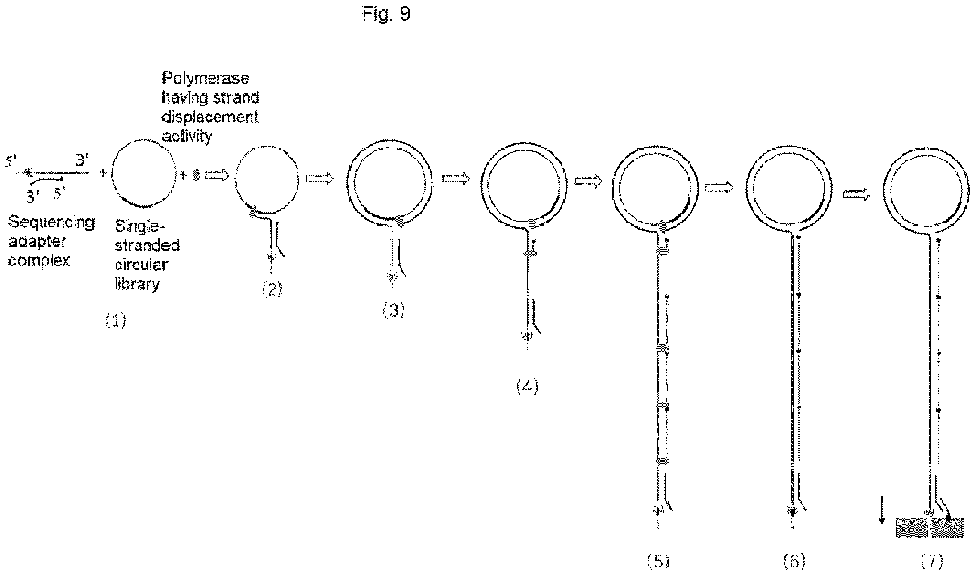

In some implementation solutions, there is a sequence that can terminate polymerase amplification between the first complementary sequence and the first primer sequence of the first strand of the sequencing adapter, for example, a spacer, an abasic site, or any combination thereof; the spacer of the sequence that can terminate polymerase amplification is iSp18, iSp9, iSpC3, iSpC6, iSpC12, or any combination thereof; the number of the sequences that can terminate polymerase amplification is 1-10; and the artificially modified nucleotide of the second strand of the sequencing adapter complex is not mandatory, and details on how to realize multiple amplification on the target nucleic acid sequence and nanopore sequencing are referred to Fig. 9.

For ease of operation and product production, in some implementation solutions, the individual constraining sequence may be not required, hydrophobic molecule modification may be directly performed on the 3' end of the second strand of the sequencing adapter, and the hydrophobic molecule is selected from any one or more of the following: a lipid, a fatty acid, a sterol, a carbon nanotube, a peptide, a protein, and an amino acid, for example, cholesterol, palmitate, or tocopherol.

In some implementation solutions, the unwinding direction of the helicase is the 5'-3' direction; the helicase is selected from any one or more of the following: a Dda helicase, a Pif 1 helicase, an XPD helicase, a T7Gp41 helicase, and a DnaB helicase; and the selected helicase may be subjected to mutation modification.

A typical implementation of the present disclosure further provides a method for multiple amplifications of a target nucleic acid sequence. The method includes the following steps. At S1, a sequencing adapter complex, a circular library, and a polymerase having strand displacement activity are bound.

At S2, rolling circle amplification is performed on the circular library, and a reverse primer is introduced simultaneously, where the reverse primer and a first primer sequence are reversely complementary to each other, and complementarily bind to a single strand obtained by rolling circle amplification of the circular library, so as to realize rolling circle amplification and two-strand synthesis of the target nucleic acid sequence circular library, thereby completing amplification of a target nucleic acid sequence.

The circular library may be a single-stranded circular library, or may also be a double-stranded circular library. The 5' end of the reverse primer includes the artificially modified nucleic acid sequence that can inhibit polymerase strand displacement activity. Preferably, an artificially modified nucleotide in the artificially modified nucleic acid sequence is one or more of LNA, PNA, and BNA; and more preferably, the number of the artificially modified nucleotides in the artificially modified nucleic acid sequence is 2-10. Preferably, the reverse primer is excessively added.

In the present disclosure, typically, S1 includes: the first strand of the sequencing adapter in the sequencing adapter complex binds to the circular library through complementary base pairing, and the polymerase uses the circular library as the template and the first strand as the primer to bind at the primer-template junction, so as to form an amplification complex; and/or S2 includes: the polymerase uses dNTP or NTP for a polymerization reaction, the first strand of the sequencing adapter extends to obtain the sequencing strand, based on the strand displacement activity of the polymerase, the sequencing strand extends by means of rolling circle amplification, the reverse primer specifically binds to the sequencing strand and extends under the action of the polymerase, so as to complete two-strand synthesis, and two-strand synthesis stops when encountering the artificially modified nucleic acid sequence of the 5' end of the previous reverse primer or the second strand of the sequencing adapter, or a sequence that is able to terminate polymerase amplification between a first complementary sequence and a first primer sequence of the first strand of the sequencing adapter, so as to complete multiple amplifications of the target nucleic acid sequence.

Preferably, a temperature for first incubation is 4 °C-37 °C, and a time is 10-600 minutes; preferably, a temperature for second incubation is 20 °C-37 °C, and a time is 10-360 minutes; and more preferably, the reaction temperature of S2 is 30 °C-37 °C, and the time is 20-360 minutes.

In some implementation solutions, the polymerase having strand displacement activity is selected from a DNA polymerase or an RNA polymerase. The selected polymerase may also be a polymerase having strand displacement activity that is obtained by modifying a polymerase without strand displacement activity, such as a Bst DNA polymerase, an SD DNA polymerase, a phi29 DNA polymerase, a Bsu Large Fragment DNA polymerase, a Klenow Fragment DNA polymerase, a T4 DNA polymerase, a T7 DNA polymerase, a DNA Polymerase I, a T3 RNA polymerase, a T7 RNA polymerase, an SP6 RNA polymerase, an *E.coli* RNA polymerase, or any combination thereof.

In some implementation solutions, the reaction buffer contains a pH buffer system, preferably, the pH buffer system is a dihydrogen phosphate-hydrogen phosphate buffer system, a carbonate-sodium bicarbonate buffer system, a Tris-HCl buffer system, a HEPES buffer system, a MOPS buffer system, or any combination thereof. Preferably, the reaction buffer contains K⁺, Na⁺, or any combination thereof; and preferably, the reaction buffer contains Mg²⁺, Mo²⁺, Cu²⁺, Fe²⁺, Zn²⁺, Ca²⁺, Pb²⁺, Cd²⁺, or any combination thereof. Preferably, the reaction buffer contains an additive or an auxiliary reagent that enhances a polymerase extension reaction, and the additive or the auxiliary reagent is dimethyl sulfoxide, glycerol, formamide, bovine serum albumin, ammonium sulphate, polyethylene glycol, gelatin, a non-ionic detergent, N,N,N-trimethylglycine, a single-stranded nucleic acid binding protein, dithiothreitol, ethylenediamine tetraacetic acid, or any combination thereof.

A typical implementation of the present disclosure provides a method for nanopore sequencing. The method includes the following steps. 1) A target product after a target nucleic acid sequence is amplified is obtained by using any one of the above methods for multiple amplifications of a target nucleic acid sequence; and 2) the target product binds to a membrane of a nanopore sequencer through a constraining sequence, where the target product is sequenced under control of a helicase, so as to sequence a target sequence. Sequencing accuracy is significantly improved by performing nanopore sequencing on the target nucleic acid sequence for the plurality of times.

In some implementation solutions, step 2) includes: a second strand of a sequencing adapter binds to the constraining sequence, and an amplification product binds to the membrane of the nanopore sequencer, or the constraining sequence on the second strand of the sequencing adapter directly binds the amplification product to the membrane of the nanopore sequencer, where a sequence length of the constraining sequence is 10-50 nucleotides; an end of the constraining sequence is modified with a hydrophobic molecule, and the hydrophobic molecule is selected from any one or more of the following: a lipid, a fatty acid, a sterol, a carbon nanotube, a peptide, a protein, and an amino acid, for example, cholesterol, palmitate, or tocopherol. A sequencing voltage is applied, then a guide sequence of the sequencing adapter is captured by a nanopore under the action of an electric field force, and the target nucleic acid sequence is sequenced for the plurality of times under the control of the helicase. Preferably, the sequencing voltage is more than 10 mV, preferably 50 mV-250 mV. For ease of operation and product production, in some implementation solutions, the individual constraining sequence may be not required, hydrophobic molecule modification may be directly performed on the 3' end of the second strand of the sequencing adapter, and the hydrophobic molecule is selected from any one or more of the following: a lipid, a fatty acid, a sterol, a carbon nanotube, a peptide, a protein, and an amino acid, for example, cholesterol, palmitate, or tocopherol.

In some implementation solutions, the nanopore is a transmembrane protein pore or a solid-state pore. In some implementation solutions, a transmembrane protein from which the transmembrane protein pore derives is selected from hemolysin, MspA, MspB, MspC, MspD, FraC, ClyA, PA63, CsgG, CsgD, XcpQ, SP1, a phi29 connector protein, InvG, GspD, or any combination thereof.

In some implementation solutions, the transmembrane pore is also linked to an additional peptide, and the additional peptide is selected from a tag, a restriction enzyme cutting site, a signal peptide or presequence, a detectable marker, or any combination thereof.

In some implementation solutions, the membrane of the nanopore sequencer is an amphiphilic membrane, a polymer membrane, or any combination thereof, and the membrane of the nanopore sequencer is a phospholipid bilayer, or a diblock copolymer, or a triblock copolymer.

In some implementation solutions, the sequencing buffer contains a pH buffer system, preferably, the pH buffer system is a dihydrogen phosphate-hydrogen phosphate buffer system, a carbonate-sodium bicarbonate buffer system, a Tris-HCl buffer system, a HEPES buffer system, a MOPS buffer system, or any combination thereof.

In some implementation solutions, the sequencing buffer contains NTP, dNTP, ddNTP, or any combination thereof.

In some implementation solutions, the sequencing buffer contains K⁺, Na²⁺, or any combination thereof.

In some implementation solutions, the sequencing buffer contains Mg²⁺, Mo²⁺, Cu²⁺, Fe²⁺, Zn²⁺, Ca²⁺, Pb²⁺, Cd²⁺, or any combination thereof.

The beneficial effects of the present disclosure are further described in detail below with reference to specific embodiments.

Embodiment 1: Single-stranded circular library construction
1. A single-stranded circular library might be constructed by using various known methods, and in this embodiment, the single-stranded circular library was prepared by connecting two ends of a double-stranded target nucleic acid to an adapter containing a hairpin structure.
2. End repair and A tailing were performed on a target nucleic acid (SEQ ID NO.1) to be tested having a signature sequence by using NEBNext FFPE DNA Repair Mix(NEB, M6630) and NEBNext Ultra II End repair/dA-tailing Module(NEB, E7546) according to manufacturer's description.
3. A hairpin sequence (SEQ ID NO.2) was dissolved with a TE buffer (pH=8) according to the manufacturer's description, and was annealed as a hairpin structure. An annealing process was to perform incubation for 5 minutes at 95 °C, to reduce a temperature to 25 °C at a cooling rate of 0.1 °C/s, and to continuously perform incubation for 30 minutes.
4. A ligation reaction was performed on the target nucleic acid to be tested that had been subjected to end repair and A tailing and the annealed adapter containing the hairpin structure by using NEBNext Quick Ligation Module(NEB, E6056) according to the manufacturer's description, and a reaction condition was to perform incubation for 10 minutes at 25 °C.
5. The single-stranded circular library was purified by using AMPure XP beads (Beckman Coulter, A63882) according to the manufacturer's description.

Embodiment 2 Sequencing adapter complex construction

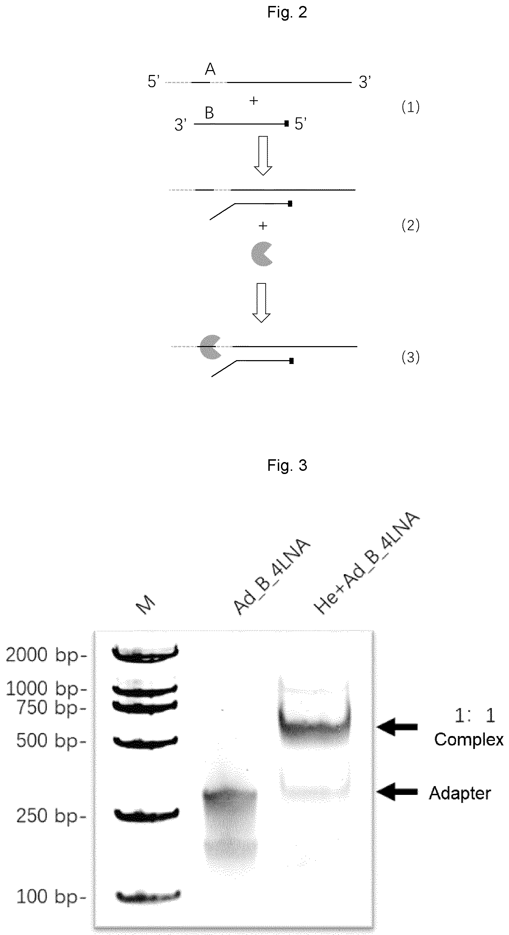

A construction process of a sequencing adapter complex is shown in Fig. 2.

In Process (1), a synthesized first strand (A, SEQ ID NO.3) and a second strand (B, SEQ ID NO.4) were annealed as a sequencing adapter according to a ratio of 1:1. The first strand sequentially included, from 5' to 3', a guide sequence (30 iSpC3s, represented with a gray dashed), a helicase binding sequence (10 Ts, represented with a black solid line), a spacer region (4 iSp18s, represented with a gray dashed), and sequences (represented with a black solid line) complementary to a second strand complementary sequence and to the single-stranded circular library; the second strand sequentially included, from 5' to 3', sequences complementary to a first strand complementary sequence and to a constraining sequence (a 5' end includes an artificially modified nucleic acid sequence that could inhibit polymerase strand displacement activity, for example, LNA, PNA, or BNA, etc., represented with a bold black solid line; and the remaining unmodified nucleic acid sequences were represented with black solid lines). In Process (2), the sequencing adapter and a helicase (light gray with a notched circle, and an unwinding direction was a direction from 5' to 3', SEQ ID NO.5) were incubated to a complex. In Process (3), a buffer containing ATP was added; due to the action of a spacer region, only 1 motor protein bound between the guide sequence and the spacer region was reserved; and the sequencing adapter complex in which a ratio of the helicase to the sequencing adapter was 1:1 was obtained through magnetic bead purification.

Specific operation steps were shown as follows.
1. SEQ ID NO.3 and SEQ ID NO.4 were respectively dissolved with the TE buffer (pH=8) according to the manufacturer's description. The SEQ ID NO.3 and the SEQ ID NO.4 were annealed as the sequencing adapter, which was named as Ad-B-4LNA, according to the ratio of 1:1. An annealing process was to perform incubation for 5 minutes at 95 °C, to reduce a temperature to 25 °C at a cooling rate of 0.1 °C/s, and to continuously perform incubation for 30 minutes.
2. Prokaryotic expression of the helicase He (T4Dda-(ΔM1)G1/E94C/C109A/C136A/K194L/A360C, SEQ ID NO.5) was completed in Escherichia coli, and a target protein was obtained through multi-step purification.
3. The helicase He and the sequencing adapter Ad-B-4LNA were mixed according to a molar ratio of 9:1, a final concentration of the reaction buffer was 25 mM HEPES, 50 mM KCI, 0.5 mM EDTA, and 2.5 mM MgCl₂, pH = 8.0, and incubation was performed for 30 minutes at room temperature.
4. 0.25 volume of 5 mM ATP was added to an incubation product, and incubation was performed for 30 minutes at room temperature.
5. The sequencing adapter complex was purified by using AMPure XP beads (Beckman Coulter, A63882) according to the manufacturer's description, so as to obtain a sequencing adapter complex He+Ad-B-4LNA. Electrophoresis detection was performed on the purified sequencing adapter complex, and results were shown in Fig. 3; and a large number of 1:1 He+Ad-B-4LNA sequencing adapter complexes were obtained (in Fig. 3, a Lane 1 was a DNA molecular weight standard (M), a Lane 2 was the sequencing adapter Ad-B-4LNA, a Lane 3 was the sequencing adapter complex He+Ad-B-4LNA, and arrows respectively pointed to the sequencing adapter complex in which a ratio of the helicase to the sequencing adapter was 1:1, and the sequencing adapter).

Embodiment 3 target nucleic acid sequence amplification and nanopore sequencing
1. Control group: the single-stranded circular library obtained in Embodiment 1, the sequencing adapter complex He+Ad-B-4LNA obtained in Embodiment 2, and the reaction buffer (NEB, M0269S) of the phi29 DNA polymerase were well mixed, and incubated for 30 minutes at 30 °C. Experimental group: the single-stranded circular library obtained in Embodiment 1, the sequencing adapter complex He+Ad-B-4LNA obtained in Embodiment 2, the phi29 DNA polymerase having strand displacement activity, and the reaction buffer of the phi29 DNA polymerase were well mixed, and incubated for 30 minutes at 30 °C.

**Table 1 Incubation system formula for S1**

| Reagent | Control group | Experimental group |
|---|---|---|
| 80 ng/uL single-stranded circular library | 2 µL | 2 µL |
| 5 ng/uL He+Ad-B-4LNA | 2 µL | 2 µL |
| 10 U/pL phi29 DNA polymerase | - | 1 µL |
| 10×phi29 DNA polymerase reaction buffer | 1 µL | 1 µL |
| H₂O | 5 µL | 4 µL |
| Total volume | 10 µL | 10 µL |

2. The dNTP was added to the control group and the experimental group, excess reverse primers (SEQ ID NO.6) were added to the incubation product, and incubation was performed for 30 minutes at 30 °C.

**Table 2 Incubation system formula for S2**

| Reagent | Control group | Experimental group |
|---|---|---|
| Product in S1 | 10 µL | 10 µL |
| 10 mM dNTP | 4 µL | 4 µL |
| 1 µM reverse primer (SEQ ID NO.6) | 5 µL | 5 µL |
| 10×phi29 DNA polymerase reaction buffer | 1 µL | 1 µL |
| Total volume | 20 µL | 20 µL |

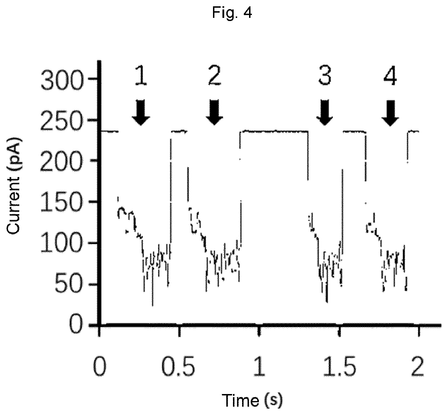

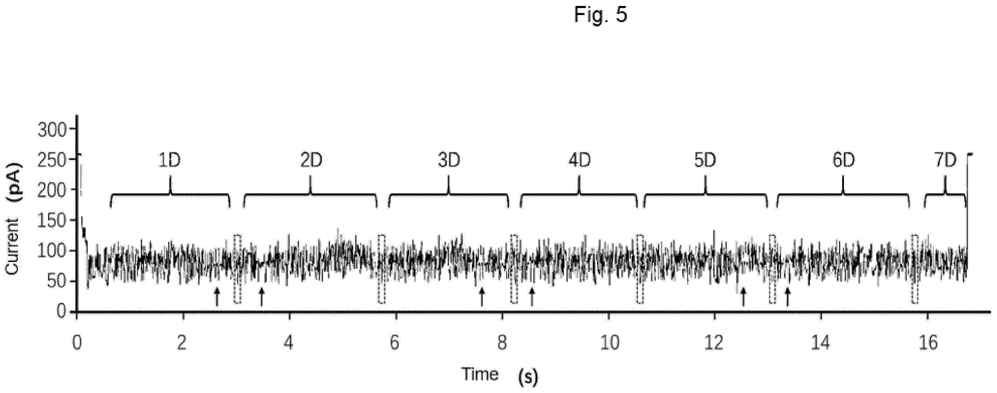

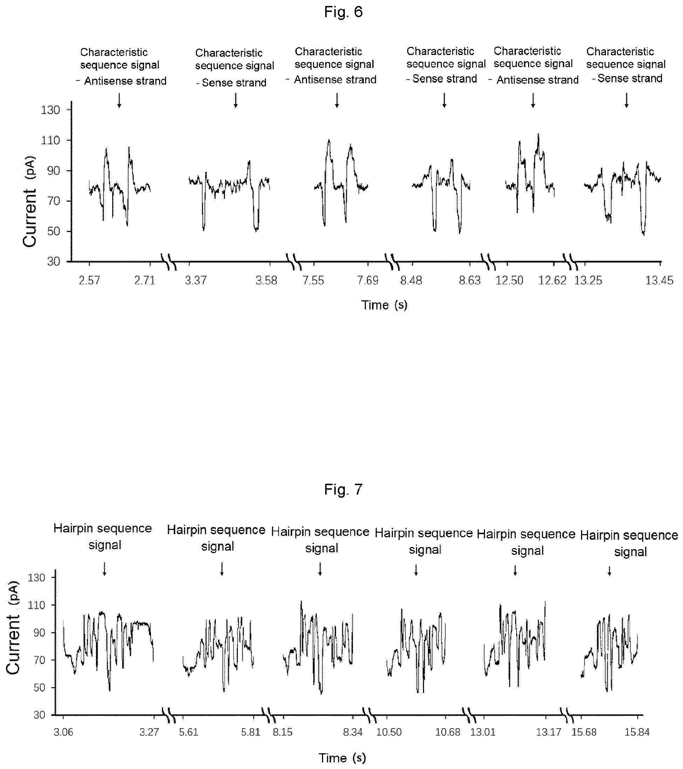

3. Reaction products in the control group and the experimental group were purified by using AMPure XP beads (Beckman Coulter, A63882) according to the manufacturer's description.
4. A single channel nanopore detection system was built based on a patch clamp and a signal amplifier, so as to complete pore embedding of a single porin.
5. In the nanopore detection system, the individual nanopore protein was inserted in the phospholipid bilayer, the reaction products in the control group and the experimental group respectively were well mixed with the constraining sequence (SEQ ID NO.7), and added to the single channel system, respectively, changes in a current signal were observed at 180 mV and obtained. The sequencing buffer included 470 mM KCI, 25 mM HEPES, 10 mM MgCl₂, and 30 mM ATP, with pH = 8.10; and a sequencing temperature was 30 °C.
6. The control group only obtained the current signal for nanopore sequencing of the sequencing adapter, and Fig. 4 showed a current signal diagram of nanopore sequencing of 4 sequencing adapters. The experimental group observed and obtained the current signal for multiple nanopore sequencing of the target nucleic acid sequence to be tested, as shown in Fig. 5, according to a characteristic sequence signal and a hairpin sequence signal, it might be determined that a target sequence was continuously sequenced for 7 times, and 1D, 2D, 3D, 4D, 5D, 6D, and 7D were respectively used for the current signals for the first, second, third, fourth, fifth, sixth, and seventh sequencing, where the seventh sequencing was only partially completed. From sequencing signal current amplitude change diagram, it might be found that durations and sequencing speeds for different times of sequencing (first, second, third, fourth, fifth, and sixth) were relatively the same. An arrow represented the characteristic sequence signal, and Fig. 6 was an enlarged view of the current signal; and a dashed rectangular box represented the hairpin sequence signal, and Fig. 7 was an enlarged view of the current signal.

Polynucleotide sequences used in the embodiments
SEQ ID NO. 1: 
5' GAATTCGCCATCAGATTGTGTTTGTTAGTCGCTTTTTTTTTTTGGAATTTTTTTTTTGGAA
TTTTTTTTTTGCGCTAACAACCTCCTGCCGTTTTGCCCGTGCATATCGGTCACGAACAAATCTG
ATTACTAAACACAGTAGCCTGGATTTGTTCTATCAGTAATCGACCTTATTCCTAATTAAATAGAGC
AAATCCCCTTATTGGGGGTAAGACATGAAGATGCCAGAAAAACATGACCTGTTGGCCGCCATTC
TCGCGGCAAAGGAACAAGGCATCGGGGCAATCCTTGCGTTTGCAATGGCGTACCTTCGCGGC
AGATATAATGGCGGTGCGTTTACAAAAACAGTAATCGACGCAACGATGTGCGCCATTATCGCCT
AGTTCATTCGTGACCTTCTCGACTTCGCCGGACTAAGTAGCAATCTCGCTTATATAACGAGCGT
GTTTATCGGCTACATCGGTACTGACTCG 3' (the underlined was the signature sequence)
SEQ ID NO. 2: 
5'Phosphorylation-TGCAATACGTAACTGAACGAAGTACATTTTTTTTCCTTGGCTCACAGAA
CGACATGGCTTTTTTTTTGTACTTCGTTCAGTTACGTATTGCAT 3'
SEQ ID NO. 3: 
5’NNNNNNNNNNNNNNNNNNNNNNNNNNNNNNTTTTTTTTTTYYYYGGTTGTTTCTGTTGGT
GCTGATATTGCTGCCATGTCGTTCTGTGAGCCAAGG 3’ (N = iSpC3, Y = iSp18)
SEQ ID NO. 4: 
5’ (LNA_A) (LNA_A) (LNA_T) (LNA_A)TCAGCACCAACAGAAACAACCTTTGAGGCGAGC
GGTCAA 3’
SEQ ID NO. 5: 
GSHGTFDDLTEGQKNAFNIVMKAIKEKKHHVTINGPAGTGKTTLTKFIIEALISTGETGIILAAPT
HAAKKILSKLSGKEASTIHSILKINPVTYECNVLFEQKEVPDLAKARVLICDEVSMYDRKLFKILLSTIP
PWATIIGIGDNKQIRPVDPGENTAYISPFFTHKDFYQCELTEVKRSNAPIIDVATDVRNGLWIYDKVVD
GHGVRGFTGDTALRDFMVNYFSIVKSLDDLFENRVMAFTNKSVDKLNSIIRKKIFETDKDFIVGEIIV
MQEPLFKTYKIDGKPVSEIIFNNGQLVRIIEAEYTSTFVKARGVPGEYLIRHWDLTVETYGDDEYYRE
KIKIISSDEELYKFNLFLGKTCETYKNWNKGGKAPWSDFWDAKSQFSKVKALPASTFHKAQGMSV
DRAFIYTPCIHYADVELAQQLLYVGVTRGRYDVFYV
SEQ ID NO.6:
   5' (LNA_C) (LNA_C) (LNA_T) (LNA_T)GGCTCACAGAACGACATGGC 3'
SEQ ID NO.7:
   5' Cholesterol-YYYYTTGACCGCTCGC 3' (Y=iSp18)

It might be seen from the above description that, in the above embodiments of the present disclosure, the following technical effects were realized.

(1) The present disclosure was simple in library construction process and high in yield, for example, the single-stranded circular library might be constructed by directly connecting the double-stranded ends of the nucleic acid to be tested to the adapter containing the hairpin structure. (2) In the present disclosure, multiple sequencing might be realized, and the number of times for sequencing was greater than 4. (3) In the present disclosure, the sequencing process had been a double-stranded sequencing process controlled by the helicase, and the speeds for multiple sequencing were relatively the same. (4) The sequencing adapter of the present disclosure was simple in design, it was easy to anneal two strands as a destination configuration, and a yield was high; (5) the present disclosure was suitable for the single-stranded circular library constructed by various manners, and even for the double-stranded circular library (which had bulges or gaps at the binding site of the first strand of the sequencing adapter complex); and (6) excess reverse primers were added during the amplification process of the present disclosure, and the generation of long single strands was reduced, thereby reducing the generation of a complex secondary structure affecting sequencing.

The above are only the preferred embodiments of the present disclosure and are not intended to limit the present disclosure. For those skilled in the art, the present disclosure may have various modifications and variations. Any modifications, equivalent replacements, improvements and the like made within the spirit and principle of the present disclosure all fall within the scope of protection of the present disclosure.

## Claims

1. A sequencing adapter, comprising a first strand and a second strand, wherein
the first strand sequentially comprises, from a 5' end to a 3' end, a sequencing guide sequence, a helicase binding sequence, a restriction structure, a first complementary sequence, and a first primer sequence, the restriction structure prevents a helicase from moving, and the first strand has a 3' free end;
the second strand sequentially comprises, from the 5' end to the 3' end, a second complementary sequence, and a constraining sequence or a sequence complementary to a nucleic acid sequence of the constraining sequence, and the constraining sequence comprises the nucleic acid sequence modified with a hydrophobic molecule at an end;
the first complementary sequence is reversely complementary to the second complementary sequence, and the sequencing adapter is formed by annealing the first strand and the second strand; and the sequencing adapter further comprises a structure that prevents the second strand from being displaced by a polymerase.

2. The sequencing adapter according to claim 1, wherein the structure that prevents the second strand from being displaced by the polymerase comprises at least one of the following: a sequence that is located on the 5' end of the second strand and inhibits polymerase strand displacement activity, or a blocking structure located between the first complementary sequence and the first primer sequence of the first strand, wherein the blocking structure prevents the nucleic acid sequence from extending through the polymerase.

3. The sequencing adapter according to claim 2, wherein the sequence that is located on the 5' end of the second strand and inhibits the polymerase strand displacement activity comprises an artificially modified nucleic acid sequence, and an artificially modified nucleotide in the artificially modified nucleic acid sequence comprises at least one of LNA, PNA, or BNA;
further, the artificially modified nucleic acid sequence comprises 2-10 artificially modified nucleotides; and/or
the blocking structure comprises at least one of a spacer region or an abasic site, the spacer region comprises a spacer, and the spacer is selected from at least one of iSp18, iSp9, iSpC3, iSpC6, or iSpC12; and
further, the blocking structure comprises 1-10 spacers or abasic sites.

4. The sequencing adapter according to claim 1, wherein the restriction structure comprises a spacer region, the spacer region comprises a spacer, and the spacer is selected from at least one of iSp18, iSp9, iSpC3, iSpC6, or iSpC12; the restriction structure comprises 2-8 spacers;
preferably, the sequencing guide sequence comprises 10-50 nucleotides or iSpC3;
preferably, the helicase binding sequence comprises 5-40 nucleotides, more preferably, the helicase binding sequence comprises 5-40 thymidylic acids;
preferably, the first complementary sequence or the second complementary sequence comprises 5-80 nucleotides; and
preferably, the nucleic acid sequence of the constraining sequence comprises 10-50 nucleotides.

5. The sequencing adapter according to claim 1, wherein the hydrophobic molecule is selected from any one or more of the following: a lipid, a fatty acid, a sterol, a carbon nanotube, a peptide, a protein, and an amino acid; and
preferably, the hydrophobic molecule is cholesterol, palmitate, or tocopherol.

6. The sequencing adapter according to any one of claims 1 to 5, wherein the first primer sequence comprises a specific primer or a random primer, and the first primer sequence is able to bind to a circular library;
preferably, the first primer sequence comprises 5-80 nucleotides;
optionally, the circular library is a single-stranded circular library or a double-stranded circular library;
optionally, the single-stranded circular library has an introduced known sequence, and the known sequence complementarily binds to the first primer sequence;
optionally, the double-stranded circular library has an introduced known sequence, and there is a bulge or gap at a complementary junction of the known sequence and the first primer sequence; and
optionally, there is no known sequence in the circular library, the first primer sequence is a degenerate primer sequence, and any sequence of the circular library complementarily binds to the degenerate primer sequence.

7. A sequencing adapter complex, comprising the sequencing adapter according to any one of claims 1 to 6 and a helicase, wherein the helicase binds to a helicase binding sequence, and unwinds in a 5'-3' direction; preferably, the helicase is selected from any one or more of the following: a Dda helicase, a Pif 1 helicase, an XPD helicase, a T7 Gp41 helicase, and a DnaB helicase; and the helicase is subjected to mutation modification.

8. A kit for multiple amplifications of a target nucleic acid sequence, comprising the sequencing adapter according to any one of claims 1 to 6; and
further, the kit further comprises a polymerase having strand displacement activity, a reaction buffer, and a helicase.

9. A kit for multiple amplifications of a target nucleic acid sequence, comprising the sequencing adapter complex according to claim 7; and
further, the kit further comprises a polymerase having strand displacement activity and a reaction buffer.

10. The kit according to claim 8 or 9, further comprising one or more of a reverse primer, dNTP or NTP, and a circular library construction-related reagent;
the reverse primer is able to complementarily bind to a single strand obtained by rolling circle amplification of a circular library; the reverse primer has a 3' free end; preferably, a 5' end of the reverse primer contains an artificially modified nucleic acid sequence that is able to inhibit polymerase strand displacement activity;
preferably, an artificially modified nucleotide in the artificially modified nucleic acid sequence is one or more of LNA, PNA, and BNA; and more preferably, the number of the artificially modified nucleotides in the artificially modified nucleic acid sequence is 2-10.

11. A method for multiple amplifications of a target nucleic acid sequence, comprising the following steps:
S1, combining the sequencing adapter complex according to claim 7, a target nucleic acid sequence circular library, and a polymerase having strand displacement activity; and
S2, performing rolling circle amplification on the circular library, and introducing a reverse primer simultaneously, wherein the reverse primer and a first primer sequence are reversely complementary to each other, and complementarily bind to a single strand obtained by rolling circle amplification of the circular library, so as to realize rolling circle amplification and two-strand synthesis of the target nucleic acid sequence circular library, thereby completing multiple amplifications of a target nucleic acid sequence.

12. The method according to claim 11, wherein the reverse primer is able to complementarily bind to a single strand obtained by rolling circle amplification of a circular library; the reverse primer has a 3' free end; a 5' end of the reverse primer contains an artificially modified nucleic acid sequence that is able to inhibit polymerase strand displacement activity;
preferably, an artificially modified nucleotide in the artificially modified nucleic acid sequence is one or more of LNA, PNA, and BNA; more preferably, the number of the artificially modified nucleotides in the artificially modified nucleic acid sequence is 2-10; and
preferably, the reverse primer is added in excess.

13. The method according to claim 12, wherein S1 comprises: a first strand of the sequencing adapter in the sequencing adapter complex binding to the circular library through complementary base pairing, and the polymerase using the circular library as a template and the first strand as a primer to bind to a primer-template junction, so as to form an amplification complex; and/or
S2 comprises: the polymerase using dNTP or NTP for a polymerization reaction, extending the first strand of the sequencing adapter to obtain a sequencing strand, based on the strand displacement activity of the polymerase, the sequencing strand extending by means of rolling circle amplification, the reverse primer specifically binding to the sequencing strand and extending under the action of the polymerase, so as to complete two-strand synthesis, and two-strand synthesis stopping when encountering the artificially modified nucleic acid sequence of the 5' end of the previous reverse primer or the second strand of the sequencing adapter, or a sequence that is able to terminate polymerase amplification between a first complementary sequence and a first primer sequence of the first strand of the sequencing adapter, so as to complete multiple amplifications of the target nucleic acid sequence.

14. The method according to claim 11, 12, or 13, wherein a reaction temperature of S1 is 4 °C-37 °C, and a time is 10-600 minutes;
preferably, a reaction temperature of S2 is 20 °C-37 °C, and a time is 10-360 minutes; and preferably, the reaction temperature of S2 is 30 °C-37 °C, and the time is 20-360 minutes.

15. The method according to claim 11, wherein the polymerase having strand displacement activity is selected from a DNA polymerase or an RNA polymerase;
optionally, the polymerase is a polymerase having strand displacement activity that is obtained by modifying a polymerase without strand displacement activity; and
preferably, the polymerase is selected from a Bst DNA polymerase, an SD DNA polymerase, a phi29 DNA polymerase, a Bsu Large Fragment DNA polymerase, a Klenow Fragment DNA polymerase, a T4 DNA polymerase, a T7 DNA polymerase, a DNA Polymerase I, a T3 RNA polymerase, a T7 RNA polymerase, an SP6 RNA polymerase, an *E.coli* RNA polymerase, or any combination thereof.

16. The method according to claim 11, wherein S1 and S2 are performed in a reaction buffer;
the reaction buffer contains a pH buffer system, preferably, the pH buffer system is a dihydrogen phosphate-hydrogen phosphate buffer system, a carbonate-sodium bicarbonate buffer system, a Tris-HCl buffer system, a HEPES buffer system, a MOPS buffer system, or any combination thereof;
preferably, the reaction buffer contains K⁺, Na²⁺, or any combination thereof;
preferably, the reaction buffer contains Mg²⁺, Mo²⁺, Cu²⁺, Fe²⁺, Zn²⁺, Ca²⁺, Pb²⁺, Cd²⁺, or any combination thereof; and
preferably, the reaction buffer contains an additive or an auxiliary reagent that enhances a polymerase extension reaction, and the additive or the auxiliary reagent is dimethyl sulfoxide, glycerol, formamide, bovine serum albumin, ammonium sulphate, polyethylene glycol, gelatin, a non-ionic detergent, N,N,N-trimethylglycine, a single-stranded nucleic acid binding protein, dithiothreitol, ethylenediamine tetraacetic acid, or any combination thereof.

17. A method for nanopore sequencing, comprising the following steps:
1) obtaining a target product after a target nucleic acid sequence is amplified by using the method for multiple amplifications of a target nucleic acid sequence according to any one of claims 11 to 16; and
2) binding the target product to a membrane of a nanopore sequencer through a constraining sequence, wherein the target product is sequenced under control of a helicase, thereby sequencing a target sequence.

18. The method according to claim 17, wherein, step 2) comprises:
a second strand of a sequencing adapter binding to the constraining sequence, and binding an amplification product to the membrane of the nanopore sequencer, or the constraining sequence on the second strand of the sequencing adapter directly binding the amplification product to the membrane of the nanopore sequencer, wherein a sequence length of the constraining sequence is 10-50 nucleotides; an end of the constraining sequence is modified with a hydrophobic molecule at an end, and the hydrophobic molecule is selected from any one or more of the following: a lipid, a fatty acid, a sterol, a carbon nanotube, a peptide, a protein, and an amino acid; preferably, the hydrophobic molecule is cholesterol, palmitate, or tocopherol; and
applying a sequencing voltage, then a guide sequence of the sequencing adapter being captured by a nanopore under the action of an electric field force, the helicase passing through a spacer region, and sequencing the target nucleic acid sequence for the plurality of times under the control of the helicase, wherein
preferably, the sequencing voltage is more than 10 mV, preferably 50 mV-250 mV.

19. The method according to claim 17 or 18, wherein the nanopore of the nanopore sequencer is a transmembrane protein pore or a solid-state pore;
preferably, a transmembrane protein from which the transmembrane protein pore derives is selected from hemolysin, MspA, MspB, MspC, MspD, FraC, ClyA, PA63, CsgG, CsgD, XcpQ, SP1, a phi29 connector protein, InvG, GspD, or any combination thereof;
optionally, the transmembrane protein is also linked to an additional peptide, and the additional peptide is selected from a tag, a restriction enzyme cutting site, a signal peptide or presequence, a detectable marker, or any combination thereof; and
optionally, the membrane of the nanopore sequencer is an amphiphilic membrane, a polymer membrane, or any combination thereof, and the membrane of the nanopore sequencer is a phospholipid bilayer, or a diblock copolymer, or a triblock copolymer.

20. The method according to any one of claims 17 to 18, wherein a sequencing buffer used during sequencing contains a pH buffer system, preferably, the pH buffer system is a dihydrogen phosphate-hydrogen phosphate buffer system, a carbonate-sodium bicarbonate buffer system, a Tris-HCl buffer system, a HEPES buffer system, a MOPS buffer system, or any combination thereof;
preferably, the sequencing buffer contains NTP, dNTP, ddNTP, or any combination thereof;
preferably, the sequencing buffer contains K⁺, Na²⁺, or any combination thereof; and
preferably, the sequencing buffer contains Mg²⁺, Mo²⁺, Cu²⁺, Fe²⁺, Zn²⁺, Ca²⁺, Pb²⁺, Cd²⁺, or any combination thereof.
